# DEMANDE DE BREVET EUROPEEN

(11) **EP 1 852 085 A1**
(43) Date de publication de la demande: **07.11.2007**
(21) Numéro de dépôt: 06113410.2
(22) Date de dépôt: 03.05.2006
(51) Int. Cl.: A61C 1/14, A61B 17/16, B23B 31/20

(54) **Instrument à main à usage dentaire ou chirurgical**

(71) Demandeur: Bien-Air Holding SA, 2504 Bienne (CH)
(72) Inventeur: Mosimann, David, 2503, Bienne (CH); Maitre, Luc, 2885, Epauvillers (CH)
(74) Mandataire: GLN

(57) **Abrégé**

L'invention concerne un instrument à main, à usage dentaire ou chirurgical, destiné à entraîner en rotation un outil rectiligne (13) amovible. Il comporte :
• un fourreau tubulaire (1),
• un arbre creux (3) monté rotatif à l'intérieur du fourreau et constitué :
o d'une partie amont (5), et
o d'une partie aval (6) comportant elle-même une partie amont (11) enserrant partiellement la partie amont (5) de l'arbre et une partie aval (12) dont le diamètre interne correspond sensiblement au diamètre externe de l'outil (13),

• une pince (14) solidaire en rotation de l'arbre (3) mais déplaçable selon son axe et disposée à l'intérieur de la partie amont (5) de l'arbre, concentriquement à elle, et
• des moyens pour déplacer la pince (14) longitudinalement de manière à ce que ses mors (17) pénètrent sous la partie amont (5) de l'arbre (3) et assurent ainsi le serrage de l'outil.

## Description

La présente invention se rapporte au domaine des instruments médicaux. Elle concerne, plus particulièrement, un instrument à main destiné à entraîner en rotation, à grande vitesse, un outil rectiligne amovible dont l'extrémité constitue une fraise ou organe analogue utilisable dans les cabinets ou laboratoires dentaires ainsi qu'en microchirurgie.

Un instrument de ce type est décrit, par exemple, dans le document WO 2005/089666. Typiquement, la queue de l'outil est maintenue par une pince disposée à l'intérieur d'un arbre rotatif creux entraîné à son extrémité par un moteur électrique ou une turbine à air et monté par des roulements dans un fourreau tubulaire fixe. Le serrage et le déserrage de la pince se fait en tournant dans un sens ou l'autre un manchon monté rotatif sur le fourreau.

Afin de faciliter la vision du champ opératoire, les instruments à main à usage dentaire ou microchirurgical nécessitent, bien évidemment, un long nez effilé. Une telle contrainte limite hélas la possibilité d'assurer un bon guidage de l'outil à l'extrémité du nez au moyen d'un très petit roulement suffisamment résistant.

La queue de l'outil, dont le diamètre n'est généralement que de 2,35 mm, étant d'une rigidité limitée et pouvant présenter un « mal-rond », la pince qui assure son serrage risque donc de la forcer quelque peu, ce qui provoque son arquage et l'oscillation de l'extrémité active de l'outil. On comprendra aisément que ce flottement intempestif est très nuisible à la précision d'action de la fraise.

La présente invention a notamment pour but de fournir un instrument dans lequel le bruit et les vibrations, résultant d'un serrage et d'un guidage imparfaits de la queue de l'outil, sont très fortement réduits, voire éliminés. L'invention a aussi pour but de moins solliciter le roulement à billes et donc d'en améliorer la durée de vie.

De façon plus précise, l'invention concerne un instrument à main, à usage dentaire ou chirurgical, destiné à entraîner en rotation, à grande vitesse, un outil rectiligne amovible dont l'extrémité constitue une fraise ou organe analogue. Cet instrument comporte :
- un fourreau tubulaire ayant un axe longitudinal,
- un arbre creux, de même axe, monté rotatif à l'intérieur dudit fourreau et constitué d'une partie amont et d'une partie aval comportant elle-même une partie amont enserrant partiellement la partie amont de l'arbre et une partie aval dont le diamètre interne correspond sensiblement au diamètre externe dudit outil,
- une pince solidaire en rotation dudit arbre mais déplaçable selon ledit axe et disposée à l'intérieur de la partie amont de l'arbre, concentriquement à elle, ladite pince se présentant, dans sa partie amont, sous la forme d'un arbre creux de diamètre interne correspondant sensiblement au diamètre externe de l'outil et qui, au delà, se prolonge par plusieurs mors élastiquement déformables destinés au serrage de l'outil, et
- des moyens pour déplacer ladite pince longitudinalement soit vers l'amont de manière à ce que ses mors pénètrent sous la partie amont de l'arbre et assurent ainsi le serrage de l'outil, soit vers l'aval de manière à ce que les mors échappent à ladite partie amont et assurent ainsi la desserrage de l'outil.

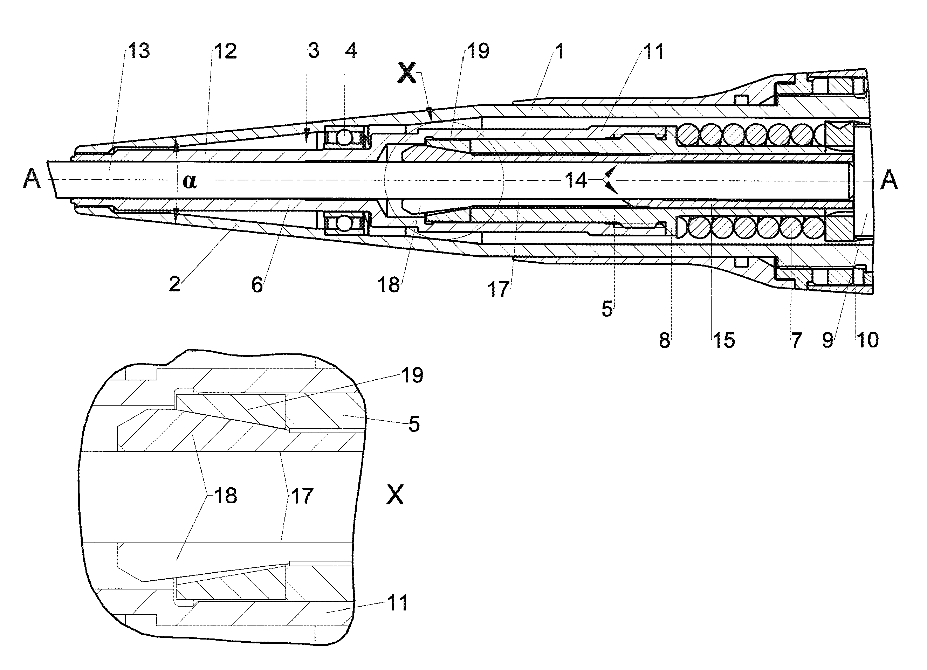

D'autres caractéristiques et avantages de la présente invention ressortiront de la description qui va suivre, faite en regard de la figure unique annexée qui est une vue partielle en coupe longitudinale d'un tel instrument.

Afin de faciliter la lecture, la présente description utilisera les mots « amont » et « aval » pour désigner respectivement l'extrémité de l'instrument à partir de laquelle l'outil est entraîné en rotation (partie droite de la figure) et son extrémité portant la pointe active de l'outil (partie gauche de la figure). De plus, le dessin ne montre pas l'extrémité amont de l'instrument car celle-ci est identique à celle décrite dans le document WO 2005/089666 précité, auquel on pourra se référer pour comprendre la manière dont se fait l'entraînement de l'outil.

Sur le dessin, on a représenté en 1 un fourreau cylindrique, d'axe AA, dont le nez 2, côté aval, est effilé pour former un angle α de typiquement 5 à 10° assurant à l'opérateur la meilleure vision possible du champ opératoire.

Le fourreau 1 contient essentiellement un arbre creux 3, de même axe AA, dont l'extrémité amont est couplée à l'arbre du moteur d'entraînement. Cet arbre est monté rotatif dans le fourreau 1 grâce à un roulement à billes 4 disposé dans le nez 2, sensiblement en son milieu. Un deuxième roulement (non visible au dessin), situé à l'amont, sert à faire tourner l'arbre dans le fourreau.

L'arbre creux 3 est, en fait, constitué d'une partie amont 5 et d'une partie aval 6.

La partie amont 5 de l'arbre 3 est entourée partiellement d'un ressort 7 comprimé entre un rebord 8 formé à sa périphérie et un barreau transversal 9, axialement déplaçable, dont les diverses fonctions apparaîtront plus loin. Ce ressort a ainsi tendance à repousser le barreau 9 vers l'amont.

Le barreau 9 peut être déplacé vers l'amont ou vers l'aval dans des fentes longitudinales de l'arbre 3 (non visibles au dessin) sous l'action d'un manchon externe 10 qui peut se visser ou se dévisser sur la partie amont du fourreau1. Dans un sens de rotation du manchon, le barreau 9 progresse vers l'aval en comprimant le ressort 7 alors que, dans son autre de sens de rotation, le barreau progresse vers l'amont et déleste le ressort.

Le dessin montre que la partie aval 6 de l'arbre 3 comporte, elle même, une partie amont 11 et une partie aval 12, toutes deux cylindriques.

La partie amont 11 enserre la partie amont 5 de l'arbre 3 jusqu'à venir en butée contre le rebord 8. Sa partie aval 12 a un diamètre interne correspondant sensiblement au diamètre externe de la queue 13 de l'outil à manipuler avec l'instrument. On notera que le roulement 4 est positionné à l'extrémité amont de la partie aval 12.

Une pince 14, déplaçable longitudinalement, est disposée à l'intérieur de la partie amont 5 de l'arbre 3, concentriquement à elle. Dans sa portion amont qui s'étend sensiblement jusqu'au niveau du rebord 8 du fourreau, cette pince est sous la forme d'un arbre creux 15 de diamètre interne correspondant sensiblement au diamètre externe de la queue 13 de l'outil à manipuler. Au delà, l'arbre 15 se prolonge par trois mors de même diamètre interne et élastiquement déformables 17, distribués à 120° les uns des autres, dont l'extrémité constitue un sabot à profil conique 18 d'épaisseur croissante vers l'aval. Le barreau 9 assure la solidarisation en rotation de la partie amont 5 de l'arbre 3 avec la pince 14.

Enfin, la partie amont 5 de l'arbre 3 est prolongée, sans faire partie d'elle, par une bague indépendante 19 présentant un profil interne conique qui correspond au profil conique des trois sabots 18. Son diamètre externe constant est légèrement inférieur au diamètre interne de la partie amont 5 de l'arbre 3 afin de lui offrir un jeu qui la rend flottante entre les sabots 18 et la partie amont 11 de la partie aval 6 de l'arbre 3.

Le dessin montre l'instrument en position de travail obtenue en faisant tourner le manchon 10, dans un sens approprié, autour de la partie amont du fourreau 1 de manière à tirer le barreau 9 vers l'amont. Les trois mors 17 de la pince 14 sont ainsi également entraînés vers l'amont la pince 14 et s'insèrent dans la bague flottante 19. Avec l'aide du ressort 7, ils compriment fortement la queue 13 de l'outil qui se trouve ainsi immobilisée et peut être entraînée en rotation par l'arbre 3.

Une rotation dans l'autre sens du manchon 10 fait revenir le barreau 9 vers l'aval, faisant également glisser vers l'aval les mors 17 qui échappent à la bague 19 et, venant en butée contre l'extrémité aval de la partie amont 5 de l'arbre (position non représentée au dessin), cessent d'emprisonner la queue 13 de l'outil qui peut ainsi être extrait de l'instrument.

Ainsi est proposé un instrument de microchirurgie dont les avantages sont exposés ci-après.
1, La réalisation de l'arbre 3 en une partie amont 5 qui entoure la pince 14 et une partie aval 6 entourant partiellement la partie amont 5 de l'arbre, concentriquement à elle, et formant un canon pour le passage de la queue 13 de l'outil permet :
   - d'aligner parfaitement les axes de la pince 14 et du canon 6 ;
   - de guider parfaitement l'outil jusqu'à l'extrémité du nez 2 du fourreau;
   - de centrifuger les déchets afin d'éviter qu'ils ne pénètrent dans l'instrument.
2. Le positionnement du roulement 4 sur le canon 6, en aval de la pince, offre une excellente concentricité entre l'axe de rotation déterminé par le roulement et l'axe de l'outil.
3. La bague conique flottante 19 assurant le serrage de la pince 14 permet :
   - en cas de léger désalignement entre les axes du canon 6 et de la pince, du fait que la bague est flottante, un alignement automatique de ces deux axes sur l'axe de rotation ;
   - en cas de vibration de l'outil, du fait que la bague est flottante et auto-bloquante, d'éviter un délestage des forces de serrage.

Ainsi, l'instrument selon l'invention est doté de caractéristiques grâce auxquelles le bruit et les vibrations sont très fortement réduits, l'efficacité du serrage de l'outil est considérablement améliorée, le guidage de l'outil est parfaitement assuré et la durée de vie du roulement est nettement allongée.

Ces avantages sont particulièrement appréciables pour un instrument dont l'outil, pouvant tourner à des vitesses de l'ordre de 50 000 à 100 000 tours/minute, doit être capable de travailler, sans flottement, avec la plus grande des précisions.

## Revendications

1. Instrument à main, à usage dentaire ou chirurgical, destiné à entraîner en rotation, à grande vitesse, un outil rectiligne (13) amovible dont l'extrémité constitue une fraise ou organe analogue, **caractérisé en ce qu'**il comporte :
• un fourreau tubulaire (1) ayant un axe longitudinal (AA),
• un arbre creux (3), de même axe, monté rotatif à l'intérieur dudit fourreau et constitué :
o d'une partie amont (5), et
o d'une partie aval (6) comportant elle-même une partie amont (11) enserrant partiellement la partie amont (5) de l'arbre et une partie aval (12) dont le diamètre interne correspond sensiblement au diamètre externe dudit outil (13),
• une pince (14) solidaire en rotation dudit arbre (3) mais déplaçable selon ledit axe (AA) et disposée à l'intérieur de la partie amont (5) de l'arbre, concentriquement à elle, ladite pince se présentant, dans sa partie amont, sous la forme d'un arbre creux (15) de diamètre interne correspondant sensiblement au diamètre externe de l'outil (13) et qui, au delà, se prolonge par plusieurs mors (17) élastiquement déformables destinés au serrage de l'outil, et
• des moyens pour déplacer ladite pince (14) longitudinalement soit vers l'amont de manière à ce que ses mors (17) pénètrent sous la partie amont (5) de l'arbre (3) et assurent ainsi le serrage de l'outil, soit vers l'aval de manière à ce que les mors échappent à ladite partie amont et assurent ainsi la desserrage de l'outil.

2. Instrument selon la revendication 1, **caractérisé en ce que** l'extrémité desdits mors (17) constitue un sabot à profil conique (18) d'épaisseur croissante vers l'aval et **en ce que** la partie amont (5) de l'arbre (3) est, en fait, constituée, sans faire partie d'elle, d'une une bague indépendante (19) présentant un profil interne conique qui correspond au profil conique des sabots (18), le diamètre externe constant de cette bague étant légèrement inférieur au diamètre interne de la partie amont (5) de l'arbre (3) afin de lui offrir un jeu qui la rend flottante entre les sabots (18) et la partie amont (11) de la partie aval (6) de l'arbre (3).

3. Instrument selon l'une des revendications 1 et 2, **caractérisé en ce que** ledit fourreau cylindrique (1) présente un nez effilé (2) formant un angle (α) de 5 à 10° assurant à l'opérateur la meilleure vision possible du champ opératoire.

4. Instrument selon l'une des revendications 1 à 3, **caractérisé en ce que** ledit arbre creux (3) est monté rotatif à l'intérieur du fourreau (1) à l'aide d'un roulement (4) situé, dans le nez (2) du fourreau, en aval de la pince (14), vers l'extrémité amont de la partie aval (12) de la partie aval (6) de l'arbre (3).

5. Instrument selon l'une des revendications 1 à 4, **caractérisé en ce que** lesdits moyens comportent un manchon (10) se vissant ou se dévissant sur la partie amont du fourreau (1) et assurant le déplacement longitudinal de la pince (14).
